# EUROPEAN PATENT APPLICATION

(11) **EP 3 466 411 A1**
(43) Date of publication of application: **10.04.2019**
(21) Application number: 17806767.4
(22) Date of filing: 31.05.2017
(51) Int. Cl.: A61K 9/16, A61K 9/20, A61K 9/48, A61K 31/10, A61K 31/192, A61K 31/196, A61K 31/216, A61K 31/4422, A61K 31/495, A61K 31/64, A61K 47/10, A61K 47/26, A61K 47/34, A61K 47/38

(54) **METHOD FOR PRODUCING PHARMACEUTICAL COMPOSITION CONTAINING MICROPARTICLES OF SPARINGLY-SOLUBLE DRUG**

(30) Priority: 31.05.2016 JP 2016109393
(71) Applicant: Kyowa Hakko Kirin Co., Ltd., Tokyo 100-8185 (JP)
(72) Inventor: YAMAZOE, Yohei, Tokyo 100-8185 (JP); YAMAOKA, Toshiki, Tokyo 100-8185 (JP); IWATA, Kenji, Tokyo 100-8185 (JP)
(74) Representative: Vossius & Partner Patentanwälte Rechtsanwälte mbB
(86) International application number: PCT/JP2017/020357
(87) International publication number: WO 2017/209216

(57) **Abstract**

The present invention provides a method for producing a pharmaceutical composition containing fine particles of a poorly soluble drug, comprising mixing a sugar or a sugar alcohol with a dispersion of nanoparticles of the poorly soluble drug and granulating the obtained mixture.

## Description

### Technical Field

The present invention relates to a method for producing a pharmaceutical composition containing fine particles of a poorly soluble drug. The present invention also relates to a pharmaceutical composition containing fine particles of a poorly soluble drug, and an oral pharmaceutical composition containing the pharmaceutical composition.

### Background Art

As a method for improving absorbency in a living body of a poorly soluble drug, micronization of the drug is employed. This method is an approach made in expectation that a dissolution rate is to be improved by micronizing the drug to a nano-order size or a size close to a nano-order size to dramatically increase its surface area. It is also known that nanoparticulation of a drug reduces the influence of a meal on absorbency, and this method is expected to be a useful method for improving the pharmacokinetic aspect of a poorly soluble drug.

As a method for micronizing a poorly soluble drug, a wet-milling technique is widely employed.

Patent Literature 1 discloses a method in which a poorly water-soluble pharmaceutical is ground to a size of 10 µm or less, the resultant is homogeneously dispersed in a binding solution, and the thus obtained suspension is sprayed for granulation onto a sugar and/or a sugar alcohol flowing in a fluidized bed granulation dryer.

Non Patent Literature 1 discloses a method, to be employed for miconazole and itraconazole, in which a suspension containing hydroxypropyl cellulose (HPC), sodium lauryl sulfate (SDS) and water is wet-milled using a high energy bead mill, mannitol or crystalline cellulose is added to and mixed with the thus obtained wet-milled solution (an average particle size of the drug (based on volume): about 200 to 800 nm), and the resultant suspension is powdered by spray drying or freeze drying.

Non Patent Literature 2 discloses a method, to be employed for glibenclamide, in which the drug precedently dispersed in a solvent together with a dispersant (dioctyl sodium sulfosuccinate) and subjected to spray drying is used as a starting material to obtain a suspension by adding sodium lauryl sulfate (SDS) and water thereto, the suspension is wet-milled using a high-pressure homogenizer, mannitol is added to and mixed with the thus obtained wet-milled solution (an average particle size of the drug: about 200 nm), and the resultant suspension is powdered by high-shear granulation, spray drying or freeze drying.

### Citation List

### Patent Literature

Patent Literature 1: Japanese Patent Laid-Open Publication No. H7-126154

### Non Patent Literature

Non Patent Literature 1: International Journal of Pharmaceutics 443 (2013), 209-220
Non Patent Literature 2: European Journal of Pharmaceutical Sciences 49 (2013), 565-577

### Summary of Invention

### Technical Problem

Patent Literature 1 does not, however, describe a result on re-dispersibility of drug fine particles in the composition, for example, a comparison in a particle size distribution of the drug fine particles before and after solidification with the solidified composition dispersed (re-dispersed) in water again. Besides, in a formulation produced by the technique disclosed in Patent Literature 1, the amount of the sugar and/or the sugar alcohol added to the drug is large, and hence it is apprehended that the size may be large when tableted or a formulation dose may be increased because the drug content is small.

According to Non Patent Literature 1, in a system using itraconazole as a poorly soluble drug, the solidified composition is re-dispersed in water again to make a comparison in the particle size distribution of the drug fine particles before and after the solidification, and the solidified composition obtained after the solidification exhibited satisfactory re-dispersibility. On the other hand, in a system using a powder obtained from a suspension containing crystalline cellulose, a powder obtained by the freeze drying, or miconazole as a poorly soluble drug, expected re-dispersibility is not obtained, and it can be said there is a problem in versatility of the aptitude of the drug or the production method.

In Non Patent Literature 2, the solidified composition is tableted together with various excipients (crospovidone, magnesium stearate, light anhydrous silicic acid and lactose hydrate), and the dissolution property of the thus obtained tablet in a pH 7.4 phosphate buffer is evaluated. Although relative merits in the dissolution property depending on the amount of mannitol added and the production method are argued therein, a result on the re-dispersibility of the drug fine particles in the composition is not mentioned, and no evaluation is made on an effect of improving the dissolution property at the same level as a wet-milled solution.

A problem to be solved by the present invention is to provide a pharmaceutical composition of a poorly soluble drug having excellent re-dispersibility.

### Solution to Problem

As a result of earnest studies, the present inventors have found that a pharmaceutical composition of a poorly soluble drug having excellent re-dispersibility can be obtained by mixing a sugar or a sugar alcohol with a dispersion of nanoparticles of the poorly soluble drug and granulating the obtained mixture, leading to accomplishment of the present invention.

The present invention relates to the following (1) to (12):
(1) A method for producing a pharmaceutical composition containing fine particles of a poorly soluble drug, the production method comprising mixing a sugar or a sugar alcohol with a dispersion of nanoparticles of the poorly soluble drug and granulating the obtained mixture.
(2) The production method described in (1), wherein the sugar or the sugar alcohol is at least one selected from erythritol, xylitol, sorbitol and sucrose.
(3) The production method described in (1), wherein the sugar or the sugar alcohol is at least one selected from erythritol, xylitol and sucrose.
(4) The production method described in any one of (1) to (3), wherein the nanoparticles of the poorly soluble drug are nanoparticles of the poorly soluble drug obtained by wet milling.
(5) The production method described in any one of (1) to (4), wherein a particle size distribution (D50) of the nanoparticles is 2 µm or less.
(6) The production method described in any one of (1) to (5), wherein granulating is performed by a wet granulation method.
(7) The production method described in any one of (1) to (6), wherein the dispersion of the nanoparticles of the poorly soluble drug contains a surfactant and/or a polymer.
(8) The production method described in any one of (1) to (7), wherein an aqueous solution of the sugar or the sugar alcohol is mixed.
(9) A pharmaceutical composition containing fine particles of a poorly soluble drug, obtained by the production method described in any one of (1) to (8).
(10) The pharmaceutical composition described in (9), wherein the composition is in the form of a granule.
(11) An oral pharmaceutical composition, comprising the pharmaceutical composition containing fine particles of a poorly soluble drug described in (9) or (10), and an excipient.
(12) The oral pharmaceutical composition described in (11), wherein the composition is in the form of a tablet, a capsule or a granule.

### Advantageous Effects of Invention

According to the present invention, a pharmaceutical composition of a poorly soluble drug having excellent re-dispersibility can be provided.

### Description of Embodiments

The present invention relates to a production method for a pharmaceutical composition containing fine particles of a poorly soluble drug, in which a sugar or a sugar alcohol is mixed with a dispersion of nanoparticles of the poorly soluble drug and the obtained mixture is granulated.

In the present invention, a poorly soluble drug means a drug defined with a term of soluble, sparingly soluble, slightly soluble, very slightly soluble, or practically insoluble or insoluble among drugs defined with terms relating solubility of very soluble, freely soluble, soluble, sparingly soluble, slightly soluble, very slightly soluble, and practically insoluble, or insoluble in the Japanese Pharmacopoeia 16th edition.

The poorly soluble drug is not especially limited, and examples thereof include probucol, mefenamic acid, fenofibrate, flurbiprofen, cinnarizine, nifedipine and glibenclamide.

In the present invention, the dispersion of the nanoparticles of the poorly soluble drug can be prepared by any of known methods by which a dispersion can be obtained.

The dispersion of the nanoparticles of the poorly soluble drug may be obtained by adding the poorly soluble drug to a dispersion medium to be crudely dispersed therein and wet milling the thus obtained crude dispersion, or the dispersion of the nanoparticles of the poorly soluble drug may be obtained by precedently dry milling the poorly soluble drug and adding the resultant to a dispersion medium.

The nanoparticles of the poorly soluble drug contained in the dispersion are preferably nanoparticles of the poorly soluble drug obtained by wet milling.

An apparatus to be used for the wet milling is not especially limited, and examples thereof include a high-pressure homogenizer and a mill.

The dispersion medium to be used for dispersing the poorly soluble drug is not especially limited, water or a water-soluble medium of a lower alcohol or the like is preferably used, and water, particularly purified water is suitably used.

A concentration of the poorly soluble drug in the dispersion is not especially limited, and in terms of a mass ratio to the dispersion, is, for example, 30% or less, and preferably 20% or less.

In the present invention, the poorly soluble drug is present in the form of nanoparticles in the dispersion.

A particle size distribution of the poorly soluble drug in the dispersion is, in terms of D50, preferably 2 µm or less and more preferably 0.05 to 1 µm.

In the present invention, D50 means a particle size with which a powder is divided into two groups respectively having a larger size and a smaller size than that particle size and having equivalent amounts, and is also designated as a medium diameter.

D50 refers to a particle size corresponding to accumulation of 50% in a particle size distribution.

In the present invention, D10, D50 and D90 can be measured by a method described in an example.

The dispersion may contain, as another component in addition to the poorly soluble drug, a surfactant and/or a polymer from the viewpoint of improving dispersibility of the poorly soluble drug.

The surfactant and/or the polymer are not especially limited, and examples thereof include polysorbate 80 (Tween 80), sodium lauryl sulfate (SDS), Poroxamer, hydroxypropyl cellulose (HPC), polyvinyl pyrrolidone

(PVP), hypromellose (HPMC), methylcellulose (MC) and polyvinyl alcohol (PVA).

In the present invention, Tween 80, SDS or HPC is preferably used as the surfactant and/or the polymer.

In preparing the dispersion of the nanoparticles of the poorly soluble drug, the poorly soluble drug may be added to a solution or a suspension obtained by adding the surfactant and/or the polymer to the dispersion medium, or the poorly soluble drug may be first added to the dispersion medium with the surfactant and/or the polymer added thereto afterward.

When the dispersion of the nanoparticles of the poorly soluble drug contains the surfactant and/or the polymer, a mass ratio between the poorly soluble drug and the surfactant and/or the polymer in the dispersion is, in terms of poorly soluble drug/surfactant/polymer, preferably 1/1/1 to 1/0.05/0.05, and more preferably 1/0.1/0.3.

A sugar or a sugar alcohol is mixed with the dispersion of the nanoparticles of the poorly soluble drug to obtain a mixture containing the nanoparticles of the poorly soluble drug and the sugar or the sugar alcohol.

The sugar or the sugar alcohol is not especially limited, and examples thereof include lactose, mannitol, erythritol, xylitol, sorbitol and sucrose.

The sugar or the sugar alcohol to be used may be in a D-form or an L-form. Alternatively, a DL mixture thereof in an arbitrary component ratio may be used.

From the viewpoint of re-dispersibility, the sugar or the sugar alcohol is preferably at least one selected from erythritol, xylitol, sorbitol and sucrose, and more preferably at least one selected from erythritol, xylitol and sucrose, and sucrose is suitably used.

In the present invention, a term "to use at least one sugar or sugar alcohol" has the same meaning as that one sugar or sugar alcohol may be used and two or more sugars or sugar alcohols may be used.

As the sugar or the sugar alcohol, a sugar or a sugar alcohol excluding mannitol can be used, and a sugar or a sugar alcohol excluding lactose can be used in the present invention.

In the present invention, when, for example, erythritol, xylitol, sorbitol, sucrose or the like is used, the amount of the sugar or the sugar alcohol added to the poorly soluble drug can be reduced.

The sugar or the sugar alcohol may be directly mixed with the dispersion of the nanoparticles of the poorly soluble drug, or may be mixed in the form of a solution of the sugar or the sugar alcohol, and a method for mixing the sugar or the sugar alcohol with the dispersion of the nanoparticles of the poorly soluble drug is not especially limited. Besides, in the present invention, in obtaining a mixture by mixing the sugar or the sugar alcohol with the dispersion of the nanoparticles of the poorly soluble drug, the method does not exclude mixing the dispersion of the nanoparticles of the poorly soluble drug with a solution of the sugar or the sugar alcohol.

The sugar or the sugar alcohol can be mixed in the form of a solution of the sugar or the sugar alcohol, and a solvent of the sugar or the sugar alcohol is not especially limited, water or a water-soluble solvent such as a lower alcohol is preferably used, and water, particularly purified water is suitably used.

A concentration of the sugar or the sugar alcohol in the solution is not especially limited.

A mass ratio between the poorly soluble drug and the sugar or the sugar alcohol in the mixture is, in terms of poorly soluble drug/(sugar or sugar alcohol), preferably 1/20 to 1/3, and more preferably 1/10 to 1/3.

When a content of the sugar or the sugar alcohol is 20 parts by mass or less with respect to 1 part by mass of the poorly soluble drug, a resultant tablet can be made small and drug content can be increased.

The mass ratio between the poorly soluble drug and the sugar or the sugar alcohol in the mixture approximates a mass ratio of the fine particles of the poorly soluble drug in the pharmaceutical composition obtained after granulation. Accordingly, the mass ratio between the poorly soluble drug and the sugar or the sugar alcohol in the mixture may directly accord with the mass ratio between the poorly soluble drug and the sugar or the sugar alcohol in the pharmaceutical composition of the fine particles of the poorly soluble drug. To the contrary, in the present invention, the mass ratio between the poorly soluble drug and the sugar or the sugar alcohol in the pharmaceutical composition of the fine particles of the poorly soluble drug can be regarded as the mass ratio between the poorly soluble drug and the sugar or the sugar drug in the mixture.

A necessary granulating component may be added to the mixture. As the necessary granulating component, a known granulating component generally used can be used.

The mixture is granulated to produce the pharmaceutical composition containing the fine particles of the poorly soluble drug.

A method for granulating the mixture containing at least the nanoparticles of the poorly soluble drug and the sugar or the sugar alcohol is not especially limited, and a known granulation method can be employed.

The present invention was accomplished through finding that aggregation of a micronized poorly soluble drug can be inhibited during granulation by allowing a sugar or a sugar alcohol to coexist in a mixture containing nanoparticles of the poorly soluble drug used for the granulation.

The pharmaceutical composition of the poorly soluble drug can be produced without losing an effect of increasing the surface area of the poorly soluble drug present in the form of nanoparticles.

Examples of the granulation method employed in the present invention include wet granulation methods such as a fluidized bed granulation method and a high-shear granulation method, and the fluidized bed granulation method is suitably employed.

Besides, as the pharmaceutical composition containing the fine particles of the poorly soluble drug, a dry sample may be obtained by removing a moisture content by drying the mixture with an oven or the like. In the present invention, the pharmaceutical composition embraces a dry sample obtained by removing a moisture content by drying the mixture.

A content of the poorly soluble drug in the pharmaceutical composition containing the fine particles of the poorly soluble drug is not especially limited, and is, in terms of a mass ratio to the pharmaceutical composition, for example, 20%, and preferably 10% or less.

A production method for the pharmaceutical composition containing the fine particles of the poorly soluble drug of the present invention is preferably a production method for a pharmaceutical composition containing the fine particles of the poorly soluble drug in which the sugar or the sugar alcohol is mixed with the dispersion of the nanoparticles of the poorly soluble drug obtained by the wet milling and the obtained mixture is granulated (whereas mannitol is excluded from the sugar or the sugar alcohol), and an aqueous solution of the sugar or the sugar alcohol is suitably added to be mixed with the nanoparticles of the poorly soluble drug, and besides, granulation is suitably performed by the fluidized bed granulation. In the present invention, the pharmaceutical composition is suitably obtained in the form of a granule. The granule may be a pharmaceutical composition obtained by the fluidized bed granulation.

The pharmaceutical composition obtained by the production method of the present invention is in the form of particles having excellent re-dispersibility.

When the poorly soluble drug and the sugar or the sugar alcohol are caused to coexist in the dispersion of the nanoparticles of the poorly soluble drug to obtain the pharmaceutical composition, the sugar or the sugar alcohol functions as a carrier (hereinafter abbreviated as "MF"; "MF" is an abbreviation of a matrix former), and the nanoparticles of the poorly soluble drug does not aggregate in the MF but can be present (fixed) in a dispersed state, which probably leads to the excellent re-dispersibility.

The pharmaceutical composition obtained by the production method of the present invention has excellent re-dispersibility probably for the following reason:
There is a tendency that as a saturated solution of the sugar or the sugar alcohol has a higher viscosity, the particle size (D50 or D90) obtained by re-dispersion of the resultant pharmaceutical composition is reduced to exhibit high re-dispersibility (namely, a high aggregation inhibiting effect). At the time of the granulation, in the mixture containing the nanoparticles of the poorly soluble drug and the sugar or the sugar alcohol, the sugar or the sugar alcohol contained in a droplet is presumed to temporarily reach a saturated dissolved state immediately before solidification. It seems that a viscosity of such a droplet in the saturated dissolved state affects mobility of the nanoparticles of the poorly soluble drug contained in the droplet, and therefore, it seems that as the viscosity of the saturated solution of the sugar or the sugar alcohol is lower, the nanoparticles have high mobility and thus easily aggregate, and that as the viscosity of the saturated solution of the sugar or the sugar alcohol is higher, the nanoparticles have low mobility and thus are difficult to aggregate. It seems, however, that there is also an influence of another factor different from the viscosity of the saturated solution.

When the sugar or the sugar alcohol to be used has a low viscosity when formed in a saturated solution, the re-dispersibility is improved by increasing the addition amount, and thus, the desired aggregation inhibiting effect can be obtained.

The pharmaceutical composition obtained in the present invention can be formed as an oral pharmaceutical composition by a usual method.

The oral pharmaceutical composition is not especially limited as long as it is a formulation that can be orally administered, and in particular, it may be in the form of a powder, a fine granule, a granule, a tablet or a capsule, is preferably a tablet, a capsule or a granule, and is suitably used in the form of a tablet or a capsule.

The pharmaceutical composition obtained in the present invention suitably in the form of a granule may be filled in a capsule together with another excipient or the like to obtain a capsule to be used as a pharmaceutical composition for oral administration of the present invention, or the pharmaceutical composition suitably in the form of a granule obtained by the present invention is mixed and/or granulated together with another excipient or the like if necessary to obtain a table to be used as the pharmaceutical composition for oral administration of the present invention.

The granule obtained in the present invention may be directly used as a granule, or may be mixed with another excipient if necessary to obtain a granule.

The excipient is not especially limited, and examples thereof include a diluent, a disintegrating agent and a lubricant.

Examples of the diluent include lactose, white sugar, starch, crystalline cellulose, D-mannitol, D-sorbitol, a starch derivative (such as corn starch), a cellulose derivative, a carbonate, a phosphate and a sulfate.

Examples of the disintegrating agent include crospovidone, croscarmellose sodium, sodium carboxymethyl starch and low substituted hydroxypropyl cellulose.

Examples of the lubricant include magnesium stearate, calcium stearate, talc, glycerin monostearate and light anhydrous silicic acid.

As the excipient, for example, a colorant, a perfume or the like may be further optionally added.

As each of such excipients, one may be singly used or a combination of two or more may be used.

In the present invention, inhibition of aggregation of the nanoparticles of the poorly soluble drug during a solidification/formulation process is achieved, and a particle size/particle size distribution of the poorly soluble drug obtained when the resultant pharmaceutical composition is re-dispersed is kept at an equivalent level as the particle size/particle size distribution of the poorly soluble drug in the dispersion of the nanoparticles of the poorly soluble drug obtained before the solidification/formulation, and thus, excellent characteristics such as dissolution property improvement obtained by nanoparticulation can be exhibited also after the solidification/formulation. Such characteristics can be achieved also when formed into a capsule or a granule. Besides, also after the pharmaceutical composition obtained in the present invention is tableted, the excellent re-dispersibility of the pharmaceutical composition is retained.

### Examples

The present invention will now be specifically described with reference to examples and comparative examples, and it is noted that the present invention is not limited to the following examples.

### (Example 1)

Probucol was put in a precedently prepared hydroxypropyl cellulose (HPC-L, Nippon Soda Co., Ltd.) aqueous solution, and was crudely dispersed therein using a homogenizer (12,000 rpm, 2 minutes) to prepare a dispersion. The thus obtained dispersion was charged in a high-pressure shear-type wet mill (microfluidizer, Powrex Corp.) to perform wet milling at a processing pressure of 207 MPa for a processing time corresponding to batch processing repeated 30 times, and thus, a dispersion containing nanoparticles (hereinafter referred to as the "nanosuspenion") was prepared. The nanosuspenion was adjusted to have a composition of probucol/hydroxypropyl cellulose of 10 parts by mass/1 part by mass.

Sucrose was added to purified water as an MF, and the resultant was stirred to prepare a sucrose aqueous solution as an MF aqueous solution.

The nanosuspenion and the sucrose aqueous solution were mixed, and the resultant was stirred to prepare a mixed solution. The mixed solution was adjusted to have a composition of probucol/sucrose of 1 part by mass/15 parts by mass.

The obtained mixed solution was dispensed into another vessel, dried with an oven (90°C, 60 to 120 minutes) to remove a moisture content, and thus, a dry sample was prepared as a pharmaceutical composition.

### (Comparative Example 1)

A dry sample (containing no MF) was prepared in the same manner as in Example 1 except that a mixed solution was prepared by dispensing the nanosuspenion of probucol into another vessel to be mixed with purified water and stirring the resultant.

### (Comparative Example 2)

A complex type fluidized bed fine particle coater/granulator (SFP-1, Powrex Corp.) was used, crystalline cellulose (particles) (CP-102, Asahi Kasei Chemicals Corporation) used as a core granule was charged in a can body of the apparatus, and the nanosuspenion of probucol obtained in Example 1 was sprayed onto the core granule of the crystalline cellulose (intake air flow: 40 to 42 m³/min, intake air temperature: 70°C, spray solution flow: 10.3 to 11.4 g/min, spray air flow: 25 L/min, rotor rotation speed: 1000 to 1003 min⁻¹). After the spraying, the resultant was dried for 5 minutes with the rotor rotated at a low speed (300 min⁻¹) to prepare a layering pharmaceutical composition (containing no MF).

### (Test Example 1)

Focusing on a particle size distribution (D10, D50, D90) of a nanoparticulated drug, a particle size distribution of the drug in the nanosuspenion before solidification was compared with a particle size distribution of the drug in a re-dispersion obtained by adding each of the dry samples or the layering pharmaceutical composition obtained as described above to purified water, and thus, re-dispersibility of the nanoparticles of the drug in a formulation obtained after the solidification is evaluated to evaluate an aggregation inhibiting effect obtained when the MF is contained.

The particle size distributions in the nanosuspenions obtained before the solidification, in a re-dispersion of the dry sample obtained in each of Example 1 and Comparative Example 1, and in a re-dispersion of the layering pharmaceutical composition obtained in Comparative Example 2 were measured using a laser diffraction/scattering particle size distribution measuring apparatus (Microtrac, Nikkiso Co., Ltd.). Purified water was used as a dispersion medium, and the measurement was performed in a circulation mode. As for the dry sample obtained in each of Example 1 and Comparative Example 1, 5 mL of purified water was added to the dry sample containing about 8 mg of probucol, and the resultant was stirred for 120 minutes to prepare the re-dispersion. As for the layering pharmaceutical composition obtained in Comparative Example 2, 5 mL of purified water was added to about 160 mg of the layering pharmaceutical composition (corresponding to about 8 mg of probucol), and the resultant was stirred for 120 minutes to prepare the re-dispersion.

The particle size distributions of the drug obtained by re-dispersing the dry sample obtained in Comparative Example 1 that is equivalent to formulation employing a usual prescription/production method and the layering pharmaceutical composition obtained in Comparative Example 2 were remarkably increased as compared with the particle size distribution of the drug in the nanosuspenion obtained before the formulation, and thus, aggregation of the drug was observed before and after the formulation. On the contrary, the dry sample obtained in Example 1 containing sucrose as the MF was found to be improved in the aggregation of the drug.

Table 1 Prescription of Example 1 and Comparative Examples 1 to 2

**[Table 1]**

| | | Example 1 | Comparative Example 1 | | | Comparative Example 2 |
|---|---|---|---|---|---|---|
| | Component | Amount (mg) | | | Component | Amount (mg) |
| Nanosuspension | Probucol | 10 | 10 | Nanosuspension | Probucol | 30 |
| | HPC | 1 | 1 | | HPC | 3 |
| | Purified Water | 89 | 89 | | Purified Water | 267 |
| MF Aqueous Solution | Sucrose | 150 | 0 | Granulating Component | Crystalline Cellulose | 500 |
| | Purified Water | 250 | 400 | | | |

Table 2 Particle Size Distribution of Example 1 and Comparative Examples 1 to 2

**[Table 2]**

| | | Example 1 | | Comparative Example 1 | | Comparative Example 2 |
|---|---|---|---|---|---|---|
| Particle Size Distribution (µm) | Nanosuspension | Re-dispersion | Nanosuspension | Re-dispersion | Nanosuspension | Re-dispersion |
| D10 | 0.24 | 0.50 | 0.25 | 2.7 | 0.41 | 9.3 |
| D50 | 0.51 | 1.6 | 0.52 | 23 | 0.79 | 30 |
| D90 | 0.98 | 5.7 | 0.96 | 84 | 1.6 | 66 |

### (Examples 2 to 7)

Each of various drugs (fenofibrate, nifedipine, glibenclamide, flurbiprofen, cinnarizine and mefenamic acid) was weighed in a zirconia vessel, a hydroxypropyl cellulose (HPC-SSL, Nippon Soda Co., Ltd.)/Tween 80 (Wako Pure Chemical Industries Ltd.) aqueous solution having been prepared in a prescribed concentration was subsequently added thereto to obtain a suspension, a zirconia ball (a zirconia grinding ball, YTZ, diameter: 0.1 mm, Nikkato Corporation) was put therein, and the vessel was covered with a lid. The wet milling was performed using a planetary centrifugal nano pulverizer (NP-100, Thinky Corporation), and thereafter, purified water was added to the resultant for dilution, and the zirconia ball was removed through a screen to prepare a nanosuspenion. The nanosuspenion was adjusted to have a composition of drug/HPC-SSL/Tween 80 of 10 parts by mass/3 parts by mass/1 part by mass.

In all the nanosuspenions thus prepared, the particle size distribution of the drug in terms of D50 was about 0.20 µm.

Sucrose was added to purified water as the MF, and the resultant was stirred to prepare a sucrose aqueous solution as an MF aqueous solution.

Each of the nanosuspenions of the various drugs was mixed with the sucrose aqueous solution, and the resultant was stirred to prepare a mixed solution. The mixed solution was adjusted to have a composition of drug/sucrose of 1 part by mass/10 parts by mass.

Each of the obtained mixed solutions was dispensed into another vessel, dried (90°C, 60 to 120 minutes) with an oven to remove a moisture content, and thus, a dry sample was prepared as a pharmaceutical composition.

### (Comparative Examples 3 to 8)

Dry samples (containing no MF) were prepared respectively in the same manner as in Examples 2 to 7 except that the nanosuspenions of the various drugs were not mixed with the sucrose aqueous solution to prepare mixed solutions, namely, each of the nanosuspenions of the various drugs were dispensed into another vessel and merely dried (90°C, 60 to 120 minutes) with an oven.

### (Test Example 2)

A particle size distribution of the drug in the nanosuspenion obtained before the solidification was compared with a particle size distribution of the drug in a re-dispersion obtained by adding each of the dry samples obtained as described above to purified water, and thus, the re-dispersibility of the nanoparticles of the drug in a formulation obtained after the solidification was evaluated to evaluate the aggregation inhibiting effect achieved when the MF was contained. The D50 of the drugs in the nanosuspenions obtained before the solidification was all about 0.20 µm.

This test was performed in the same manner as in Test Example 1 except that a re-dispersion was prepared by adding purified water to obtain a drug concentration of 20 mg/mL when re-dispersed and mixing the resultant by inverting five times.

With respect to all the drugs, a dry sample containing the MF was found to be improved in the re-dispersibility of the drug as compared with a dry sample containing no MF. A particularly remarkable aggregation inhibiting effect was exhibited with respect to flurbiprofen. Besides, with respect to nifedipine and glibenclamide, the D50 obtained when re-dispersed was improved by the MF addition to about 0.20 µm, which is equivalent to that of the nanosuspenion obtained before the solidification.

Table 3 Prescription of Examples 2 to 7 and Comparative Examples 3 to 8

**[Table 3]**

| | | Example 2 | Comparative Example 3 | Example 3 | Comparative Example 4 | Example 4 | Comparative Example 5 | Example 5 | Comparative Example 6 | Example 6 | Comparative Example 7 | Example 7 | Comparative Example 8 |
|---|---|---|---|---|---|---|---|---|---|---|---|---|---|
| | | Fenofibrate | | Nifedipine | | Glibenclamide | | Flurbiprofen | | Cinnarizine | | Mefenamic Acid | |
| | Component | Amount (mg) | | | | | | | | | | | |
| Nanosuspension | Poorly Soluble Drug | 10 | 10 | 200 | 200 | 200 | 200 | 200 | 200 | 200 | 200 | 200 | 200 |
| | HPC | 3 | 3 | 60 | 60 | 60 | 60 | 60 | 60 | 60 | 60 | 60 | 60 |
| | Purified Water +Tween80 | 87 | 87 | 1740 | 1740 | 1740 | 1740 | 1740 | 1740 | 1740 | 1740 | 1740 | 1740 |
| MF Aqueous Solution | Sucrose | 100 | 0 | 2000 | 0 | 2000 | 0 | 2000 | 0 | 2000 | 0 | 2000 | 0 |
| | HPC | 7 | 7 | 140 | 140 | 140 | 140 | 140 | 140 | 140 | 140 | 140 | 140 |
| | Purified Water | 293 | 393 | 2400 | 2400 | 2400 | 2400 | 2400 | 2400 | 2400 | 2400 | 2400 | 2400 |

Table 4 Particle Size Distribution of Examples 2 to 7 and Comparative Examples 3 to 8

**[Table 4]**

| | Example 2 | Comparative Example 3 | Example 3 | Comparative Example 4 | Example 4 | Comparative Example 5 | Example 5 | Comparative Example 6 | Example 6 | Comparative Example 7 | Example 7 | Comparative Example 8 |
|---|---|---|---|---|---|---|---|---|---|---|---|---|
| | Fenofibrate | | Nifedipine | | Glibenclamide | | Flurbiprofen | | Cinnarizine | | Mefenamic Acid | |
| Particle Size Distribution (µm) | Re-dispersion | Re-dispersion | Re-dispersion | Re-dispersion | Re-dispersion | Re-dispersion | Re-dispersion | Re-dispersion | Re-dispersion | Re-dispersion | Re-dispersion | Re-dispersion |
| D10 | 1.5 | 3.0 | 0.11 | 0.15 | 0.13 | 0.17 | 2.7 | 21 | 5.6 | 5.0 | 0.21 | 0.25 |
| D50 | 3.6 | 25 | 0.21 | 0.40 | 0.28 | 0.43 | 8.4 | 78 | 24 | 25 | 3.2 | 4.7 |
| D90 | 31 | 210 | 0.47 | 20 | 1.2 | 1.2 | 45 | 220 | 97 | 56 | 6.4 | 19 |

### (Examples 8 to 10)

Nanosuspenions were prepared in the same manner as in Example 2 except that fenofibrate, mefenamic acid and flurbiprofen were respectively used as the drug.

In all the nanosuspenions, the particle size distribution of the drug in terms of D50 was about 0.20 µm.

Sucrose used as an MF and a granulation binder (HPC-SSL) were added to purified water, and the resultant was stirred to prepare a sucrose aqueous solution as an MF aqueous solution.

The nanosuspenion of each drug and the sucrose aqueous solution were mixed, and the resultant was stirred to prepare a drug binder. The drug binder was adjusted to have a composition of drug/sucrose of 1 part by mass/10 parts by mass.

A fluidized bed granulator (FL-Labo, Freund Corp.) was used to spray the drug binder onto a granulation component (lactose hydrate) (intake air flow: 0.1 to 0.2 m³/min, intake air temperature: 75 to 85°C, spray solution flow: 1.2 to 2.5 g/min, spray air pressure: 0.15 MPa). After the spraying, the resultant was dried to prepare a pharmaceutical composition.

### (Comparative Examples 9 to 11)

Pharmaceutical compositions (containing no MF) were prepared respectively in the same manner as in Examples 8 to 10 except that the nanosuspenions of the various drugs were respectively mixed with a granulation binder aqueous solution obtained by adding the granulation binder (HPC-SSL) alone to purified water and stirring the resultants, and the resultants were stirred to prepare drug binders.

### (Test Example 3)

A particle size distribution of each drug in the nanosuspenion obtained before solidification was compared with a particle size distribution of the drug in a re-dispersion obtained by adding each of the dry samples obtained as described above to purified water, and thus, the re-dispersibility of the nanoparticles of the drug in a formulation obtained after the solidification is evaluated to evaluate the aggregation inhibiting effect achieved when the MF was contained.

This test was performed in the same manner as Test Example 1 except that a re-dispersion was prepared by adding 2 mL of purified water to about 500 mg of each pharmaceutical composition (corresponding to 10 mg of the drug), and mixing the resultant by inverting five times.

With respect to all the drugs, a pharmaceutical composition containing the MF had a remarkably small value of the particle size distribution as compared with a pharmaceutical composition containing no MF, and thus, the aggregation of the nanoparticles of the drug is inhibited during the solidification and formulation, and the re-dispersibility of the drug was found to be excellent. With respect to mefenamic acid and flurbiprofen, the D50 obtained when re-dispersed was improved by the MF addition to about 0.20 µm, which is equivalent to that of the nanosuspenion obtained before the solidification, and thus, very high re-dispersibility was exhibited.

Table 5 Prescription of Examples 8 to 10 and Comparative Examples 9 to 11

**[Table 5]**

| | | Example 8 | Comparative Example 9 | Example 9 | Comparative Example 10 | Example 10 | Comparative Example 11 |
|---|---|---|---|---|---|---|---|
| | | Fenofibrate | | Mefenamic Acid | | Flurbiprofen | |
| | Component | Amount (mg) | | | | | |
| Nanosuspension | Drug | 3 | 3 | 3 | 3 | 3 | 3 |
| | HPC | 0.9 | 0.9 | 0.9 | 0.9 | 0.9 | 0.9 |
| | Tween80 | 0.3 | 0.3 | 0.3 | 0.3 | 0.3 | 0.3 |
| | Purified Water | 26 | 26 | 26 | 26 | 26 | 26 |
| MF Aqueous Solution | Sucrose | 30 | 0 | 30 | 0 | 30 | 0 |
| | HPC | 2.1 | 2.1 | 2.1 | 2.1 | 2.1 | 2.1 |
| | Purified Water | 18.9 | 18.9 | 18.9 | 18.9 | 18.9 | 18.9 |
| Granulating Component | Lactose Hydrate | 113.7 | 143.7 | 113.7 | 143.7 | 113.7 | 143.7 |

Table 6 Particle Size Distribution of Examples 8 to 10 and Comparative Examples 9 to 11

**[Table 6]**

| | | Example 8 | Comparative Example 9 | | Example 9 | Comparative Example 10 | | Example 10 | Comparative Example 11 |
|---|---|---|---|---|---|---|---|---|---|
| | Fenofibrate | | | Mefenamic Acid | | | Flurbiprofen | | |
| Particle Size Distribution (µm) | Nanosuspension | Re-dispersion | Re-dispersion | Nanosuspension | Re-dispersion | Re-dispersion | Nanosuspension | Re-dispersion | Re-dispersion |
| D10 | 0.11 | 0.13 | 0.45 | 0.11 | 0.11 | 0.20 | 0.094 | 0.11 | 0.22 |
| D50 | 0.19 | 0.29 | 1.1 | 0.19 | 0.20 | 0.52 | 0.15 | 0.19 | 0.73 |
| D90 | 0.41 | 0.87 | 2.9 | 0.43 | 0.50 | 2.1 | 0.24 | 0.46 | 2.5 |

### (Examples 11 to 15)

Pharmaceutical compositions were prepared in the same manner as in Example 8 except that various sugars or sugar alcohols were used as the MF.

### (Test Example 4)

A particle size distribution of a drug in the nanosuspenion obtained before solidification was compared with a particle size distribution of the drug in a re-dispersion obtained by adding each of the pharmaceutical compositions obtained as described above to purified water, and thus, the re-dispersibility of the nanoparticles of the drug in a formulation obtained after the solidification is evaluated to evaluate the aggregation inhibiting effect depending on the type of the MF in the same manner as in Test Example 3.

With respect to each pharmaceutical composition containing, as the MF, D-mannitol, erythritol, xylitol, sorbitol or sucrose, the particle size distribution had a remarkably small value as compared with that of the pharmaceutical composition containing no MF (Comparative Example 9), and thus, the aggregation of the nanoparticles of the drug was inhibited during the solidification/formulation, and the re-dispersibility of the drug was found to be excellent.

Table 7 Prescription of Examples 11 to 14 and Example 8

**[Table 7]**

| | | Example 11 | Example 12 | Example 13 | Example 14 | Example 8 |
|---|---|---|---|---|---|---|
| | Component | Mannitol | Erythritol | Xylitol | Sorbitol | Sucrose |
| Nanosuspension | Fenofibrate | 3 | 3 | 3 | 3 | 3 |
| | HPC | 0.9 | 0.9 | 0.9 | 0.9 | 0.9 |
| | Tween80 | 0.3 | 0.3 | 0.3 | 0.3 | 0.3 |
| | Purified Water | 26 | 26 | 26 | 26 | 26 |
| MF Aqueous Solution | D-Mannitol | 30 | | | | |
| | Erythritol | | 30 | | | |
| | Xylitol | | | 30 | | |
| | Sorbitol | | | | 30 | |
| | Sucrose | | | | | 30 |
| | HPC | 2.1 | 2.1 | 2.1 | 2.1 | 2.1 |
| | Purified Water | 172.9 | 93.4 | 18.9 | 18.9 | 18.9 |
| Granulating Component | Lactose Hydrate | 113.7 | 113.7 | 113.7 | 113.7 | 113.7 |

Table 8 Particle Size Distribution of Examples 11 to 14 and Example 8

**[Table 8]**

| | | Example 11 | Example 12 | Example 13 | Example 14 | Example 8 |
|---|---|---|---|---|---|---|
| | | Mannitol | Erythritol | Xylitol | Sorbitol | Sucrose |
| Particle Size Distribution (µm) | Nanosuspension | Re-dispersion | Re-dispersion | Re-dispersion | Re-dispersion | Re-dispersion |
| D10 | 0.11 | 0.21 | 0.16 | 0.15 | 0.20 | 0.13 |
| D50 | 0.19 | 0.53 | 0.39 | 0.36 | 0.51 | 0.29 |
| D90 | 0.41 | 2.6 | 1.1 | 1.2 | 2.2 | 0.87 |

### (Examples 15 to 18)

Pharmaceutical compositions were prepared in the same manner as in Example 8 except that a lactose hydrate or sucrose was used as the MF and the drug binder was adjusted to have a composition of drug/MF of 1 part by mass/10 parts by mass or 1 part by mass/20 parts by mass in a system using a lactose hydrate, and drug/MF of 1 part by mass/1 part by mass, 1 part by mass/3 parts by mass or 1 part by mass/10 parts by mass in a system using sucrose.

### (Test Example 5)

A particle size distribution of a drug in the nanosuspenion obtained before solidification was compared with a particle size distribution of the drug in a re-dispersion obtained by adding each of the pharmaceutical compositions obtained as described above to purified water, and thus, the re-dispersibility of the nanoparticles of the drug in a formulation obtained after the solidification is evaluated to evaluate the aggregation inhibiting effect depending on the amount of the MF added in the same manner as in Test Example 3.

In a pharmaceutical composition containing the lactose hydrate as the MF, the re-dispersibility was improved at a ratio of drug/MF of 1 part by mass/20 parts by mass, and thus, the aggregation inhibiting effect during the solidification/formulation was observed.

In a pharmaceutical composition containing the sucrose as the MF, the aggregation inhibiting effect was exhibited even when the amount of the MF added was changed.

Table 9 Prescription of Examples 15 to 18 and Example 8

**[Table 9]**

| | | Example 15 | Example 16 | Example 17 | Example 18 | Example 8 |
|---|---|---|---|---|---|---|
| | | Lactose Hydrate | Lactose Hydrate | Sucrose | Sucrose | Sucrose |
| | Component | 1:10 | 1:20 | 1:1 | 1:3 | 1:10 |
| Nanosuspension | Fenofibrate | 3 | 2 | 3 | 3 | 3 |
| | HPC | 0.9 | 0.6 | 0.6 | 0.9 | 0.9 |
| | Tween80 | 0.3 | 0.2 | 0.3 | 0.3 | 0.3 |
| | Purified Water | 26 | 17.3 | 26 | 26 | 26 |
| MF Aqueous Solution | Lactose Hydrate | 30 | 39 | | | |
| | Sucrose | | | 3 | 9 | 30 |
| | HPC | 2.1 | 2.1 | 2.1 | 2.1 | 2.1 |
| | Purified Water | 172.9 | 237.6 | 18.9 | 18.9 | 18.9 |
| Granulating Component | Lactose Hydrate | 113.7 | 105.8 | 140.7 | 134.7 | 113.7 |

Table 10 Particle Size Distribution of Examples 15 to 18 and Example 8

**[Table 10]**

| | | Example 15 | Example 16 | Example 17 | Example 18 | Example 8 |
|---|---|---|---|---|---|---|
| | | Lactose Hydrate | Lactose Hydrate | Sucrose | Sucrose | Sucrose |
| | | 1:10 | 1:20 | 1:1 | 1:3 | 1:10 |
| Particle Size Distribution (µm) | Nanosuspension | Re-dispersion | Re-dispersion | Re-dispersion | Re-dispersion | Re-dispersion |
| D10 | 0.11 | 0.38 | 0.15 | 0.38 | 0.17 | 0.13 |
| D50 | 0.19 | 1.1 | 0.32 | 0.95 | 0.41 | 0.29 |
| D90 | 0.41 | 3.8 | 0.95 | 2.6 | 1.4 | 0.87 |

### (Example 19)

The pharmaceutical composition obtained in Example 8, crystalline cellulose (CEORUS PH-702, Asahi Kasei Chemicals Corporation) and croscarmellose sodium (Ac-di-sol, FMC) were manually mixed (100 times) in a glass bottle. Thereafter, magnesium stearate (Parteck LUB MST, Merck) was added thereto, and the resultant was manually mixed (100 times) to obtain a granule for tableting.

The granule for tableting was weight to 220 mg per tablet, and was tableted at a tableting pressure of 3 kN by using an 8 mmφ tableting pestle in an angular plate shape and a simple tableting machine (HANDTAB 200, Ichihashi Seiki Co., Ltd.) to prepare a tablet (Example 19) .

### (Comparative Example 12)

A granulation binder in which HPC-SSL, Tween 80 and sorbitol were dissolved was spray-added to an unground drug substance of a drug (fenofibrate) and a lactose hydrate corresponding to granulation components using a fluidized bed granulator (FL-Labo, Freund Corp.), and the resultant was dried to obtain a pharmaceutical composition (usual granule) (Comparative Example 12).

### (Test Example 6)

A bulk powder of the model drug (fenofibrate), the pharmaceutical compositions (Example 8, Comparative Example 9 and Comparative Example 12) and the tablet (Example 19) were evaluated for a dissolution property. A test was performed by a paddle method at 50 rpm on each of these samples taken in an amount corresponding to 9 mg of the active ingredient, using 500 mL of a 0.05% Tween 80 aqueous solution as a test solution. 5, 10, 15, 30, 60, 90 and 120 minutes after starting the dissolution test, 5 mL of an eluate was taken out to be filtered through a cellulose acetate membrane filter having a pore size of 0.2 µm or less. An initial portion of 4 mL of the filtrate was removed, and a following portion of the filtrate was used as a sample solution. After collecting the eluate, another 5 mL of the test solution was taken and added to the test solution held in a vessel. Separately, about 10 mg of the active ingredient for quantitative determination was precisely weighed, dissolved in a water/acetonitrile mixture (1:1), and irradiated with ultrasonic waves for 10 minutes, and the amount was adjusted accurately to 100 mL. 10 mL of this solution was accurately measured, and the water/acetonitrile mixture (1:1) was added thereto accurately up to an amount of 50 mL, and the resultant was used as a standard solution. Each of the sample solution and the standard solution was taken accurately in an amount of 10 µL and subjected to a test by liquid chromatography, and thus, a dissolution concentration was measured based on a peak area of fenofibrate in each of the solutions. The number n of tested samples was 1 to 2.

The dissolution property of the drug bulk powder was 1 µg/mL or less even at the time point of 120 minutes, and thus the dissolution property was very low. On the contrary to the drug bulk powder, the pharmaceutical composition (Comparative Example 12) obtained by the usual formulation prescription and production method had a dissolution property of about 4 µg/mL at the time point of 30 minutes, and thus the dissolution property was found to be improved. Probably, the wettability of fenofibrate was improved through the formulation, and hence the dissolution property was improved. With respect to the dissolution property of the pharmaceutical composition obtained by forming the drug bulk powder into a nanosuspenion and then solidifying and formulating the suspension, the pharmaceutical composition containing no MF (Comparative Example 9) showed a profile equivalent to that of the pharmaceutical composition (Comparative Example 12) obtained by the usual formulation prescription and production method. On the contrary, with respect to the pharmaceutical composition using sucrose as the MF (Example 8), a dissolved concentration was as high as about 6 µg/mL at the time point of 5 minutes, and the dissolution property was thus higher than that of the pharmaceutical composition obtained by the usual formulation prescription and production method (Comparative Example 12), which suggested a dissolution property improving effect owing to nanoparticulation of the drug.

Besides, with respect to the dissolution property of the tablet (Example 19) obtained by tableting the pharmaceutical composition obtained in Example 8, a dissolved concentration at the initial stage of the dissolution test was lower than that of the pharmaceutical composition owing to rate-limiting by a disintegration time of the tablet in a dissolution vessel, but as the tablet was disintegrated, the dissolved concentration was increased, and also the tablet (Example 19) using sucrose as the MF showed the dissolution property equivalent to that of the pharmaceutical composition (Example 8) at the time point of 30 minutes.

Table 11 Prescription of Examples 8 and 19 and Comparative Examples 9 and 12

**[Table 11]**

| | | Example 8 | Example 19 | Comparative Example 9 | Comparative Example 12 |
|---|---|---|---|---|---|
| | | Granule | Tablet | Granule | Granule |
| | Component | Composition (%) | | | |
| Granule Component | Fenofibrate | 2 | 1.39 | 2 | 2 |
| | HPC | 2 | 1.39 | 2 | 3 |
| | Tween80 | 0.2 | 0.14 | 0.2 | 0.02 |
| | Sucrose | 20 | 13.9 | | |
| | Sorbitol | | | | 20 |
| | Lactose Hydrate | 75.8 | 52.68 | 95.8 | 74.98 |
| Excipient | Crystalline Cellulose | | 25 | | |
| | Croscarmellose Sodium | | 5 | | |
| | Magnesium Stearate | | 0.5 | | |

Table 12 Dissolved Concentration in Dissolution Test of Examples 8 and 19 and Comparative Examples 9 and 12

**[Table 12]**

| | | Example 8 | Example 19 | Comparative Example 9 | Comparative Example 12 |
|---|---|---|---|---|---|
| Time (min) | Bulk Powder | Granule | Tablet | Granule | Granule |
| 0 | 0 | 0 | 0 | 0 | 0 |
| 5 | 0 | 6.02 | 3.05 | 3.62 | 2.28 |
| 10 | 0.05 | 6.78 | 5.47 | 4.18 | 3.09 |
| 15 | 0.09 | 6.68 | 6.04 | 4.35 | 3.58 |
| 30 | 0.14 | 6.77 | 6.69 | 4.48 | 3.85 |
| 60 | 0.34 | 6.66 | 5.66 | 4.34 | 4.14 |
| 90 | 0.52 | 6.78 | 5.91 | 4.38 | 4.16 |
| 120 | 0.85 | 6.95 | 6.43 | 4.11 | 3.85 |

## Claims

1. A method for producing a pharmaceutical composition containing fine particles of a poorly soluble drug, comprising mixing a sugar or a sugar alcohol with a dispersion of nanoparticles of the poorly soluble drug and granulating the obtained mixture.

2. The method according to claim 1, wherein the sugar or the sugar alcohol is at least one selected from erythritol, xylitol, sorbitol and sucrose.

3. The method according to claim 1, wherein the sugar or the sugar alcohol is at least one selected from erythritol, xylitol and sucrose.

4. The method according to any one of claims 1 to 3, wherein the nanoparticles of the poorly soluble drug are nanoparticles of the poorly soluble drug obtained by wet milling.

5. The method according to any one of claims 1 to 4, wherein a particle size distribution (D50) of the nanoparticles is 2 µm or less.

6. The method according to any one of claims 1 to 5, wherein granulating is performed by a wet granulation method.

7. The method according to any one of claims 1 to 6, wherein the dispersion of the nanoparticles of the poorly soluble drug contains a surfactant and/or a polymer.

8. The method according to any one of claims 1 to 7, wherein an aqueous solution of the sugar or the sugar alcohol is mixed.

9. A pharmaceutical composition containing fine particles of a poorly soluble drug, obtained by the method according to any one of claims 1 to 8.

10. The pharmaceutical composition according to claim 9, wherein the composition is in the form of a granule.

11. An oral pharmaceutical composition comprising the pharmaceutical composition containing fine particles of a poorly soluble drug according to claim 9 or 10, and an excipient.

12. The oral pharmaceutical composition according to claim 11, wherein the composition is in the form of a tablet, a capsule or a granule.
